Europäisches Patentamt

European Patent Office

Office européen des brevets

(11) Veröffentlichungsnummer: **0 285 223**
**A2**

(12) **EUROPÄISCHE PATENTANMELDUNG**

(21) Anmeldenummer: 88200600.0

(22) Anmeldetag: 30.03.88

(51) Int. Cl.⁴ **G01N 23/04**

(30) Priorität: 01.04.87 DE 3710936

(43) Veröffentlichungstag der Anmeldung:
**05.10.88 Patentblatt 88/40**

(84) Benannte Vertragsstaaten:
**DE FR GB IT SE**

(71) Anmelder: **Philips Patentverwaltung GmbH**
**Wendenstrasse 35 Postfach 10 51 49**
**D-2000 Hamburg 1(DE)**

(84) **DE**

(71) Anmelder: **N.V. Philips' Gloeilampenfabrieken**
**Groenewoudseweg 1**
**NL-5621 BA Eindhoven(NL)**

(84) **FR GB IT SE**

(72) Erfinder: **Harding, Geoffrey, Dr.**
**Franzosenkoppel 110a**
**D-2000 Hamburg 53(DE)**

(74) Vertreter: **Hartmann, Heinrich, Dipl.-Ing. et al**
**Philips Patentverwaltung GmbH**
**Wendenstrasse 35 Postfach 10 51 49**
**D-2000 Hamburg 1(DE)**

(54) **Röntgengerät.**

(57) Die Erfindung bezieht sich auf ein Röntgengerät mit einer einen Untersuchungsbereich an verschiedenen Positionen mit einem Primärstrahl geringen Querschnitts durchstrahlenden im wesentlichen monochromatischen Strahlenquelle, einer ersten ortsauflösenden Detektoranordnung, die die im Primärstrahl elastisch gestreute Strahlung jenseits des Untersuchungsbereichs mißt, und mit Mitteln zur Rekonstruktion eines Bildes des durchstrahlten Querschnitts. Dabei wird eine Zuordnung eines Röntgenquants dadurch erreicht, daß die erste Detektoranordnung so ausgebildet ist, daß sie im wesentlichen den in ihr durch Comptonstreuung bewirkten Energieverlust der Röntgenquanten mißt, daß eine zweite ortsauflösende Detektoranordnung vorgesehen ist, daß eine Koinzidenzeinrichtung die Koinzidenz der Signale der beiden Detektoranordnungen feststellt, und daß die Rekonstruktionsmittel so ausgebildet sind, daß bei einer Koinzidenz der Detektorausgangssignale der Weg des diese Signale hervorrufenden Röntgenquants und daraus sowie aus dem in der ersten Detektoranordnug gemessenen Energieverlust die Lage des Streupunktes auf dem Primärstrahl ermittelt wird.

Fig.1

## Röntgengerät

Die Erfindung betrifft ein Röntgengerät mit einer einen Untersuchungsbereich an verschiedenen Positionen mit einem Primärstrahl geringen Querschnitts durchstrahlenden im wesentlichen monochromatischen Strahlenquelle, einer ersten ortsauflösenden Detektoranordnung, die die im Primärstrahl elastisch gestreute Strahlung jenseits des Untersuchungsbereichs mißt, und mit Mitteln zur Rekonstruktion eines Bildes des durchstrahlten Querschnitts.

Ein solches Röntgengerät ist aus der DE-OS 34 06 905 und der DE-OS 35 26 015 bekannt. Dabei wird die Tatsache ausgenutzt, daß die elastisch gestreute Strahlung (die bei dem Streuprozeß im Gegensatz zur Comptonstreuung keinen Energieverlust erfährt) in einem kleinen Streuwinkelbereich (bis ca. 12°) eine Streuwinkelabhängigkeit hat, die für das jeweilige Material, in dem die Streuung erfolgt, charakteristisch ist. Es können daher für verschiedene Winkel innerhalb dieses Winkelbereichs bzw. für verschiedene Impulsüberträge (momentum transfer) Querschnittsbilder der Streudichteverteilung im Untersuchungsbereich hergestellt werden. Diese Bilder unterscheiden sich in der Regel deutlich voneinander und geben Aufschluß über das Material in dem Querschnitt.

Das Detektorausgangssignal wird dabei bestimmt von dem Wegintegral über die Streudichte längs des durch den Primärstrahl durchsetzten Weges im Untersuchungsbereich. Aus diesem Grund kann ein Detektorsignal nicht unmittelbar einem bestimmten Punkt bzw. einem bestimmten Bereich des Weges zugeordnet werden - selbst wenn die Streuung ausschließlich in diesem Punkt bzw. in diesem Bereich des Weges erfolgt. Es ist aber in Verbindung mit Positronemissionstomographie bekannt, daß eine verbesserte Rekonstruktion (mit größerem Signal/Rauschverhältnis) möglich ist, wenn es gelingt, den Ursprung eines Quants genauer zu lokalisieren (vgl. J. Com. Ass. Tomography, 1981, Seite 227).

Aufgabe der vorliegenden Erfindung ist es daher, ein Gerät der eingangs genannten Art so auszugestalten, daß damit die Stelle zumindest näherungsweise bestimmt werden kann, von der aus ein bestimmtes Röntgenquant zu einem Detektor gestreut wird.

Diese Aufgabe wird erfindungsgemäß dadurch gelöst, daß die erste Detektoranordnung so ausgebildet ist, daß sie im wesentlichen den in ihr durch Comptonstreuung bewirkten Energieverlust der Röntgenquanten mißt, daß eine zweite ortsauflösende Detektoranordnung vorgesehen ist, daß eine Koinzidenzeinrichtung die Koinzidenz der Signale der beiden Detektoranordnungen feststellt, und daß die Rekonstruktionsmittel so ausgebildet sind, daß bei einer Koinzidenz der Detektorausgangssignale der Weg des diese Signale hervorrufenden Röntgenquants und daraus sowie aus dem in der ersten Detektoranordnung gemessenen Energieverlust die Lage des Streupunktes auf dem Primärstrahl ermittelt wird.

Die Erfindung basiert auf folgenden Überlegungen: Ein Röntgenquant, das im Primärstrahl elastisch gestreut wird und in der ersten Detektoranordnung einen Compton-Streuprozeß erfährt und danach zur zweiten Detektoranordnung gelangt, wird in der Regel von beiden Detektoranordnungen praktisch gleichzeitig registriert. Wenn demgemäß die Koinzidenzeinrichtung die Koinzidenz zweier Ausgangs signale der beiden Detektoranordnungen feststellt, kann davon ausgegangen werden, daß sie auf dasselbe Röntgenquant zurückzuführen sind.

In beiden ortsauflösenden Detektoranordnungen kann dem Röntgenquant ein bestimmter Auftreffort zugeordnet werden; die Verbindungsgerade zwischen den beiden Auftrefforten stellt den Weg dar, auf dem das Röntgenquant von der ersten zur zweiten Detektoranordnung gelangt ist. Aus dem Energieverlust, den das Röntgenquant bei der Comptonstreuung in der ersten Detektoranordnung erfährt und der in dieser Detektoranordnung gemessen wird, kann bei vorgegebener Energie der Röntgenquanten im Primärstrahl der Streuwinkel bestimmt werden, um den das Röntgenquant bei der Comptonstreuung aus seiner vorherigen Richtung abgelenkt wird. Das Röntgenquant muß daher von einem Punkt auf der Mantelfläche eines Kegels ausgegangen sein, dessen Öffnungswinkel gleich dem doppelten Streuwinkel ist, dessen Spitze im Auftreffpunkt des Röntgenquants in der ersten Detektoranordnung liegt und dessen Achse durch die Verbindungslinie der Auftreffpunkte des Röntgenquants in den beiden Detektoranordnungen gegeben ist. Da andererseits bekannt ist, daß das Röntgenquant vom Primärstrahl ausgeht, ergibt sich daraus, daß der Ausgangspunkt des Röntgenquants durch den Schnittpunkt des Primärstrahls mit dem erwähnten Kegelmantel gegeben ist.

Es sei an dieser Stelle erwähnt, daß aus Med.Phys., Vol. 10, Nr. 4, Juli/August 1983, Seiten 421 ff bereits eine Anordnung mit einer ersten Detektoranordnung und einer zweiten Detektoranordnung bekannt ist, wobei ebenfalls aus dem Energieverlust im ersten Detektor die Kegelmantelfläche bestimmt wird, von der das Quant ausgegangen sein muß; es handelt sich dabei jedoch um eine Anordnung für die Emissions-Computertomographie, bei der lediglich die Verteilung eines Radionuklides im menschlichen Körper rekonstruiert wird.

Die Erfindung wird nachstehend anhand der Zeichnung näher erläutert. Es zeigen:

Fig. 1 ein Röntgengerät nach der Erfindung in schematischer Darstellung,

Fig. 2 eine Erläuterung der geometrischen Verhältnisse bei einem derartigen Röntgengerät,

Fig. 3 ein Blockschaltbild einer zur Auswertung der Detektorausgangssignale geeigneten Einheit, und

Fig. 4 ein entsprechend ausgebildetes Schichtaufnahmegerät.

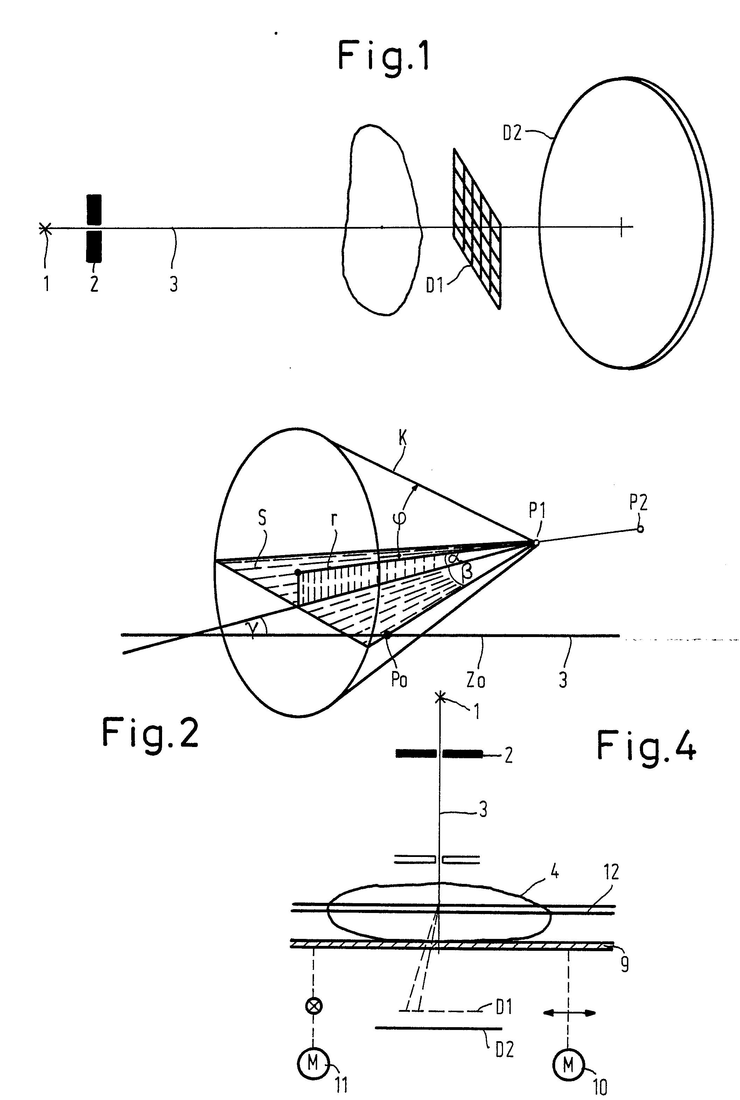

In Fig. 1 ist mit 1 eine Strahlenquelle bezeichnet, die im wesentlich monochromatische Strahlung erzeugt. Eine solche Strahlenquelle, die Strahlung lediglich mit einer bestimmten Energie bzw. in einem vergleichsweise schmalen Bereich um diese Energie herum erzeugt, kann auf verschiedene Weise hergestellt werden. Eine Möglichkeit besteht in der Verwendung eines Radionuklides. Eine andere Möglichkeit besteht in der Ausnutzung von Röntgenfluoreszenzstrahlung und eine dritte Möglichkeit besteht darin, aus dem Spektrum eines Röntgenstrahlers durch Absorptionsbandkantenfilter einen schmalen Energiebereich hervorzuheben.

Eine Blendeanordnung 2 im Strahlengang bewirkt, daß von dem Röntgenstrahlenbündel lediglich ein Primärstrahl mit geringem Querschnitt (pencil beam) 3 ein Untersuchungsobjekt 4 durchsetzt. Hinter dem Untersuchungsbereich ist eine erste Detektoranordnung D1 vorgesehen, die einerseits die Intensität des Primärstrahls, andererseits aber auch die Intensität der unter kleinen Winkeln elastisch gestreuten Streustrahlung erfaßt, wie an sich aus der deutschen Patentanmeldung P 35 26 015 bekannt. Dahinter befindet sich ein zweiter Detektor D2, der die in der ersten Detektoranordnung gestreute Röntgenstrahlung erfaßt.

Die erste Detektoranordnung besteht aus einer Anzahl von matrixartig bzw. schachbrettartig angeordneten Detektorelementen, beispielsweise 10 × 10 Detektorelementen. Die Energie der Primärstrahlung und das Material dieser Detektorelemente sind so aufeinander abgestimmt, daß die Schwächung der Röntgenstrahlung im wesentlichen durch Comptonstreuung und nur zu einem kleinen Teil durch Fotoabsorption hervorgerufen wird. Die Amplitude des Ausgangssignals eines jeden dieser Detektorelemente ist dem Energieverlust proportional, den die Röntgenquanten bei der Comptonstreuung erfahren. Es kann zwar auch vorkommen, daß Röntgenquanten in der Detektoranordnung D1 elastisch gestreut werden, doch können diese mit der Anordnung D1 nicht nachgewiesen werden, weil dabei kein Energieverlust auftritt. - Geeignete Detektorelemente bestehen aus einer etwa 10 mm starken, mit Lithium dotierten Siliziumschicht. In einem solchen Detektorelement werden bei einer Energie der Röntgenquanten im Primärstrahl von 60 keV etwa 1/3 der Röntgenquanten gestreut, und nur ein kleiner Anteil wird durch Fotoabsorption absorbiert.

Der zweite Detektor D2 ist eine konzentrisch zum Primärstrahl 3 im Abstand von etwa 10 cm zum Detektor D1 angeordnete Gammakamera, deren Detektorfläche so groß ist, daß im wesentlichen alle im Detektor D1 bis zu Streuwinkeln von 90° gestreuten Röntgenquanten darin absorbiert werden. Eine solche Gammakamera liefert bekanntlich neben einem von der Energie eines Gamma- bzw. Röntgenquants abhängigen Signal weitere vom Auftreffpunkt des Gamma quants auf der Eingangsfläche der Gammakamera abhängige Signale. Das Energieauflösungsvermögen einer solchen Gammakamera kann zur Verringerung von Störstrahlung benutzt werden. Bei einer Energie der Gammaquanten im Primärstrahl von 60 keV muß die Energie der comptongestreuten Gammaquanten nämlich zwischen etwa 50 und 60 keV liegen. Quanten, die eine wesentlich niedrigere oder höhere Energie aufweisen, können nicht aus dem Primärstrahl stammen, und die damit verbundenen Ausgangssignale werden deshalb unterdrückt.

Fig. 2 zeigt, wie bei einer Anordnung nach Fig. 1 der Punkt Po auf dem Primärstrahl 3 bestimmt werden kann, in dem ein Gammaquant elastisch gestreut wird. Dabei ist mit P1 der Punkt auf der ersten Detektoranordnung D1 bezeichnet, in dem das elastisch gestreute Röntgenquant auftrifft, und mit P2 der Punkt auf der zweiten Detektoranordnung D2, in dem das vom Punkt P1 aus durch Comptonstreuung gestreute Röntgenquant·auftrifft. Die Verbindung zwischen den Punkten P1 und P2 entspricht dem Weg des Röntgenquants zwischen den Detektoren. Die Verlängerung dieser Verbindung über den Punkt P1 hinaus ergibt eine Gerade r, die im allgemeinen nicht mit dem Primärstrahl in einer Ebene liegt. Aus dem von der Detektoranordnung D1 gemessenen Energieverlust dE des Röntgenquants läßt sich der Streuwinkel $\phi$ berechnen, unter dem das elastisch gestreute Röntgenquant bei dem Comptonstreuprozeß im Punkt P1 aus seiner vorherigen Bahn abgelenkt wird. Dafür gilt die Gleichung:

$$\phi = arc\ cos\ (1-Eo(1/(E-dE) - 1/E))\qquad(1)$$

Dabei ist arc cos die zur Kosinusfunktion inverse Funktion (cos (arc cos x) = x), Eo die Ruheenergie eines Elektrons (ca. 511 keV) und E die Energie der Röntgenquanten im Primärstrahl.

Jedes Röntgenquant, das im Punkt P1 durch einen Comptonstreuprozeß zum Punkt P2 gestreut wird und dabei um einen Winkel $\phi$ aus seiner

ursprünglichen Richtung abgelenkt wird, muß auf dem Mantel eines Kegels K liegen, dessen Spitze sich im Punkt P1 befindet, dessen Achse durch die Gerade r gegeben ist und dessen Öffnungswinkel 2 $\phi$ beträgt. Der Punkt Po, in dem der Primärstrahl 3 den Kegelmantel durchdringt, ist somit der Punkt, in dem das Röntgenquant elastisch zum Punkt P1 gestreut wurde. Die Lage dieses Punktes auf dem Primärstrahl 3, d.h. sein Abstand z0 von der Ebene der ersten Detektoranordnung D1,kann auf folgende Weise bestimmt werden: Die Ebene, die den Primärstrahl 3 und den Punkt P1 enthält, wird mit dem Kegel K geschnitten; die Schnittebene S ist in Fig. 2 schräg schraffiert. Die Schnittebene wird durch zwei Mantellinien begrenzt, von denen eine den Primärstrahl 3 innerhalb des Untersuchungsbereiches im Punkt Po schneidet. Zur Bestimmung des Schnittwinkels dieser Linie mit dem Primärstrahl 3 wird zunächst die Gerade r, die unter dem Winkel $\alpha$ zu der Schnittebene S verläuft, auf die Schnittebene projiziert; die Projektionslinie · schneidet den Primärstrahl unter einem Winkel $\gamma$. Sie unterteilt die Schnittebene S in zwei gleich große Hälften und schließt mit den beiden erwähnten Mantellinien den Winkele $\beta$ in. Dieser berechnet sich nach der Beziehung:

$$\beta = \text{arc tan } (\cos \alpha \sqrt{\tan^2\phi - \tan^2 \alpha}) \qquad (2)$$

Dabei ist arc tan die zur Tangensfunktion inverse Funktion (tan (arc tan x) = x). Damit errechnet sich der Abstand zo des Punktes Po von der Ebene der Detektoranordnung D1 zu:

$$z_0 = d/\tan(\ +\ ) \qquad (3)$$

Dabei ist d der Abstand des Punktes P1 von dem Primärstrahl 3.

Wie sich aus dem obenstehenden ergibt, kann somit jeweils der Punkt auf dem Primärstrahl 3 bestimmt werden, von dem aus das Röntgenquant elastisch zum Punkt P1 gestreut wird. Der zugehörige Streuwinkel $\beta + \gamma$ ist im allgemeinen relativ klein (kleiner als etwa 12°). Deshalb und weil der Energieverlust, den ein Röntgenquant bei dem Comptonstreuprozeß erfährt, nur relativ ungenau bestimmt werden kann (mit den heutigen Detektoren auf ca. 200 eV) ist eine exakte Lokalisierung des Ausgangspunktes des Röntgenquantes nicht möglich und ebensowenig eine exakte Bestimmung des Streuwinkels ($\beta + \gamma$), den die Bahn des elastisch gestreuten Röntgenquants mit dem Primärstrahl einschließt. Es ist jedoch möglich, bei einer Unterteilung des den Untersuchungsbereich durchsetzenden Teils des Primärstrahls in z.B. vier Abschnitte bzw. bei einer Unterteilung des Streuwinkelbereiches in einige Teilwinkelbereiche das Röntgenquant jeweils einem dieser Abschnitte bzw. Teilbereiche zuzuordnen. Bereits damit ergeben sich deutliche Verbesserungen der Rekonstruktion.

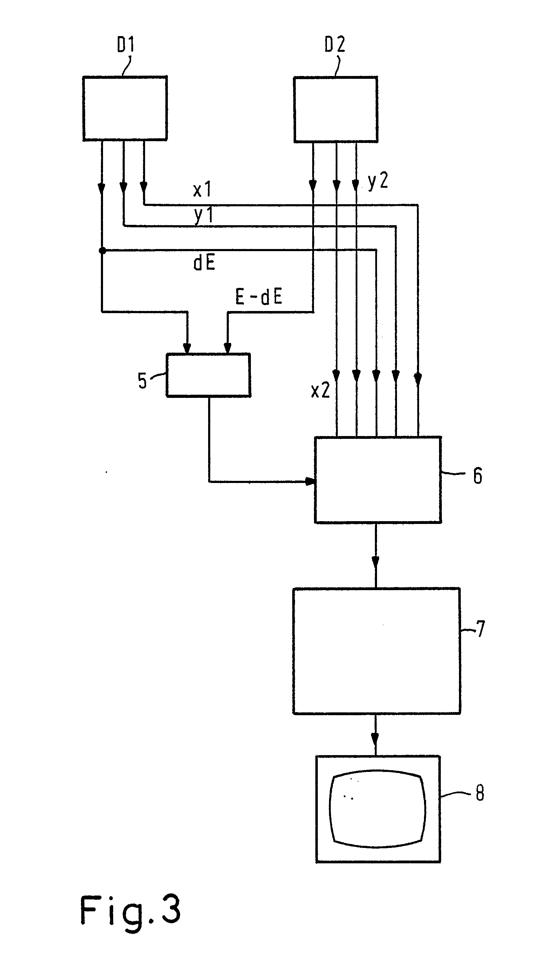

Fig. 3 zeigt ein Blockschaltbild einer in Verbindung mit der Erfindung anwendbaren Schaltung.

Die beiden Detektoren D1 und D2 liefern einerseits Signale, die dem Energieverlust dE bzw. der nach dem Comptonstreuprozeß verbleibenden Energie E-dE proportional sind. Diese Signale werden einer Koinzidenzschaltung 5 zugeführt. Außerdem liefern die beiden Detektoranordnungen D1 und D2 Signale x1, y1 bzw. x2, y2, die die Lage des Auftreffpunktes des jeweiligen Röntgenquants in einem kartesischen xy-Koordinatensystem definieren, dessen Nullpunkt beispielsweise mit dem Primärstrahl 3 zusammenfällt. Diese Signale werden zusammen mit dem vom Detektor D1 gelieferten, dem Energieverlust dE entsprechenden Signal dem Eingang einer Speicherschaltung 6 zugeführt. Diese wird durch die Koinzidenzschaltung so gesteuert, daß die Eingangswerte nur dann gespeichert werden, wenn die von den Detektoren D1 und D2 gelieferten Signale zeitgleich auftreten. Eine Rechenschaltung 7 berechnet daraus in der in Verbindung mit Fig. 2 erläuterten Weise den Streuwinkel $\beta + \gamma$ bzw. den Ausgangspunkt Po des im Primärstrahl 3 elastisch gestreuten Röntgenquants. Mit den so erhaltenen Daten wird eine Rekonstruktion der Streudichteverteilung durchgeführt, die auf einem Wiedergbegerät 8 wiedergegeben wird.

Die Rekonstruktion kann auf ähnliche Weise erfolgen wie in der deutschen Patentanmeldung P 34 06 905 in Verbindung mit der dortigen Fig. 3 geschildert; allerdings können die von der ersten Detektoranordnung gemessenen Werte von vornherein einem bestimmten Teilwinkelbereich bzw. einem bestimmten Abschnitt auf dem Primärstrahl zugeordnet werden. Dieses Rekonstruktionsverfahren setzt voraus, daß zuvor der Untersuchungsbereich vom Primärstrahl auf einer Vielzahl paralleler Strahlenpfade durchstrahlt worden ist und daß die Richtung des Strahls in bezug auf den Untersuchungsbereich um einen kleinen Winkelbetrag gedreht und danach erneut verschoben wird usw. Die Rekonstruktion mit den aufgrund dieses Verfahrens gewonnenen Meßwerten liefert Bilder der Streudichteverteilung für je einen Streuwinkel bzw. Streuwinkelbereich, was Aufschlüsse über die Zusammensetzung des Untersuchungsbereichs ergibt.

Es ist jedoch auch möglich, die zur Schichtrekonstruktion erforderlichen Daten mit Hilfe einer Abtastbewegung zu gewinnen, bei der der Untersuchungsbereich durch eine mäanderförmige, zum Primärstrahl senkrechte Verschiebebewegung abgetastet wird. Gemäß Fig. 4 kann eine Tisch platte 9, auf der sich der Patient 4 befindet, dabei mittels eines ersten Motors 10 seitlich (horizontal und in der Zeichenebene) und mittels eines zweiten Motors 11 senkrecht zur Zeichenebene verschoben werden. Ebensogut ist es aber möglich, die Anordnung 1, 2, D1 und D2 mäanderförmig in bezug auf den ruhenden Patienten zu verschieben. Ausgangssignale der Detektoranordnung D1, die jeweils dem gleichen Streuwinkel zugeordnet sind und die von Detektorelementen stammen, die vom Primärstrahl etwa chen Abstand haben, müssen dabei aus der gleichen Schicht 12 des Patienten stammen. Wie in Fig. 4 gestrichelt angedeutet, erfassen weiter vom Primärstrahl 3 entfernt liegende Detektorelemente die Streustrahlung aus der gleichen Schicht unter einem größeren Streuwinkel. Auf diese Weise ist es möglich, die Streudichteverteilung in einer (zur Zeichenebene von Fig. 4 senkrechten) Schicht 12 zu rekonstruieren - und zwar für verschiedene Streuwinkel - , ohne daß es erforderlich ist, den Primärstrahl 3 in bezug auf den Patienten 4 zu drehen.

Bei dem erfindungsgemäßen Verfahren wird nicht jedes Röntgenquant im ersten Detektor 1 durch einen Compton-Prozeß gestreut; es wird jedoch entweder im ersten oder im zweiten Detektor fotoabsorbiert. Die davon in den Detektoren hervorgerufenen Signale können in bekannter Weise (vgl. DE-OS 34 06 905 und 35 26 015) zusätzlich zur Bildrekonstruktion herangezogen werden.

**Ansprüche**

1. Röntgengerät mit einer einen Untersuchungsbereich (4) an verschiedenen Positionen mit einem Primärstrahl geringen Querschnitts durchstrahlenden, im wesentlichen monochromatischen Strahlenquelle (1), einer ersten ortsauflösenden Detektoranordnung (D1), die die im Primärstrahl elastisch gestreute Strahlung jenseits des Untersuchungsbereichs mißt, und mit Mitteln (6, 7) zur Rekonstruktion eines Bildes des durchstrahlten Querschnitts, dadurch gekennzeichnet, daß die erste Detektoranordnung (D1) so ausgebildet ist, daß sie im wesentlichen den in ihr durch Comptonstreuung bewirkten Energieverlust der Röntgenquanten mißt, daß eine zweite ortsauflösende Detektoranordnung (D2) vorgesehen ist, daß eine Koinzidenzeinrichtung (5) die Koinzidenz der Signale der beiden Detektoranordnungen (D1, D2) feststellt, und daß die Rekonstruktionsmittel (6, 7) so ausgebildet sind, daß bei einer Koinzidenz der Detektorausgangssignale der Weg (r) des diese Signale hervorrufenden Röntgenquants und daraus sowie aus dem in der ersten Detektoranordnung gemessenen Energieverlust (dE) die Lage (zo) des Streupunktes auf dem Primärstrahl (3) ermittelt wird.

2. Röntgengerät nach Anspruch 1, dadurch gekennzeichnet, daß Mittel (10, 11) zur Erzeugung einer Relativbewegung des Primärstrahls gegenüber dem Untersuchungsbereich in zwei zueinander und zur Richtung des Primärstrahls senkrechten Richtungen vorgesehen sind.

3. Vorrichtung nach Anspruch 1, dadurch gekennzeichnet, daß Mittel zur Rotation und zur translatorischen Verschiebung des Primärstrahls (3) gegenüber dem Untersuchungsbereich vorgesehen sind.

4. Vorrichtung nach einem der vorhergehenden Ansprüche, dadurch gekennzeichnet, daß die zweite Detektoranordnung (D2) eine Gammakamera ist.

5. Röntgengerät nach einem der vorhergehenden Ansprüche, dadurch gekennzeichnet, daß der erste Detektor (D1) aus einer Anzahl von matrixartig angeordneten Halbleiter-Detektorelementen besteht.

6. Röntgengerät nach Anspruch 5, dadurch gekennzeichnet, daß die Halbleiterdetektorelemente mit Lithium dotierte Siliziumdetektoren sind.

# Fig.1

# Fig.2

# Fig.4

Fig.3